# EUROPEAN PATENT APPLICATION

(11) **EP 4 847 607 A1**
(43) Date of publication of application: **02.12.2026**
(21) Application number: 25782880.6
(22) Date of filing: 28.03.2025
(51) Int. Cl.: C12N 9/02, C12N 15/77, C12P 13/08

(54) **MICROORGANISM COMPRISING NICOTINAMIDE NUCLEOTIDE TRANSHYDROGENASE VARIANT AND METHOD FOR PRODUCING L-AMINO ACID USING SAME**

(30) Priority: 04.04.2024 KR 20240046184
(71) Applicant: CJ Cheiljedang Corporation, Seoul 04560 (KR)
(72) Inventor: EOM, Ga Eul, Seoul 04560 (KR); KIM, Kyungrim, Seoul 04560 (KR); KIM, Moonjung, Seoul 04560 (KR); CHOI, Jin-Geun, Seoul 04560 (KR); HUH, Lan, Seoul 04560 (KR); KIM, Hyun Ah, Seoul 04560 (KR); SEO, Chang Il, Seoul 04560 (KR); SHIM, Jihyun, Seoul 04560 (KR)
(74) Representative: Meissner Bolte Partnerschaft mbB
(86) International application number: PCT/KR2025/004110
(87) International publication number: WO 2025/211657

(57) **Abstract**

The present disclosure relates to a variant polypeptide of a nicotinamide nucleotide transhydrogenase α-subunit; a polynucleotide encoding the variant polypeptide; a microorganism comprising the same; and use of the microorganism for producing an L-amino acid.

## Description

### [Technical Field]

The present disclosure relates to a microorganism comprising at least one selected from a variant polypeptide of a nicotinamide nucleotide transhydrogenase α-subunit, and a polynucleotide encoding the variant polypeptide; a method for producing an L-amino acid comprising culturing the microorganism in a medium; a composition for producing an L-amino acid comprising at least one selected from the microorganism, a culture of the microorganism, and a fermentation product of the microorganism; and use of the microorganism for producing an L-amino acid.

### [Background Art]

Coryneform microorganisms are gram-positive microorganisms frequently used for the industrial production of substances having various applications, such as feed, pharmaceuticals, and food products comprising L-amino acids and various nucleic acids. In recent years, diamines, keto-acids, *etc*., have also been produced from coryneform microorganisms.

In order to produce useful products through microbial fermentation, the demand for energy sources or reducing power increases alongside the demand for enhancement of the biosynthetic pathway of the target product within the microorganism. Among these, nicotinamide adenine dinucleotide phosphate (NADPH) is an essential factor in supplying reducing power. NADP⁺, the oxidized form, and NADPH, the reduced form, serve as electron carriers in the living body and are involved in various synthetic processes. Among the central metabolic pathways, NADPH is known to be primarily generated by: 1) the oxidative pentose phosphate pathway; and 2) NADP-dependent isocitrate dehydrogenase (encoded by the *Icd* gene) in the TCA cycle. In addition, various alternative pathways for supplying NADPH in various microorganisms include malate enzyme, glucose dehydrogenase, and nonphosphorylating glyceraldehyde-3-phosphate dehydrogenase.

Furthermore, enzymes that produce NADPH independently of the central metabolic pathways include transhydrogenase and ferredoxin:NADP⁺ oxidoreductase, *etc*.

As the demand for enhancement of various pathways and the demand for energy sources or reducing power coexist, there is a growing need for methods to increase the producing ability of target L-amino acids or derivatives thereof.

### [Prior Art Documents]

### [Patent Documents]

(Patent Document 1) U.S. Patent Publication US 7629142 B2

### [Disclosure]

### [Technical Problem]

The technical problem to be solved by the present disclosure is to provide a microorganism comprising a variant of a nicotinamide nucleotide transhydrogenase and a method for producing an L-amino acid using the same.

### [Technical Solution]

One aspect of the present disclosure provides a variant polypeptide of a nicotinamide nucleotide transhydrogenase α-subunit, wherein an amino acid corresponding to position 437 of SEQ ID NO: 10 is substituted with a different amino acid.

In one specific embodiment, the variant polypeptide may be one wherein the amino acid corresponding to position 437 of SEQ ID NO: 10 is substituted with alanine.

In another specific embodiment, the variant polypeptide may consist of the amino acid sequence of SEQ ID NO: 14.

In another specific embodiment, the nicotinamide nucleotide transhydrogenase α-subunit may be encoded by the *pntA* gene.

Another aspect of the present disclosure provides a nicotinamide nucleotide transhydrogenase comprising the variant polypeptide.

Still another aspect of the present disclosure provides a polynucleotide encoding the variant polypeptide.

Still another aspect of the present disclosure provides a microorganism comprising at least one selected from the variant polypeptide; a polynucleotide encoding the variant polypeptide; and a vector comprising the same.

In the microorganism according to any one of the foregoing specific embodiments, the microorganism may be a microorganism of the genus *Corynebacterium*.

In the microorganism according to any one of the foregoing specific embodiments, the microorganism of the genus *Corynebacterium* may be *Corynebacterium glutamicum*.

In the microorganism according to any one of the foregoing specific embodiments, the microorganism may have an increased L-amino acid-producing ability as compared to a microorganism comprising a polypeptide of SEQ ID NO: 10 or a polynucleotide encoding the same.

In the microorganism according to any one of the foregoing specific embodiments, the microorganism may have an increased level of L-threonine.

Still another aspect of the present disclosure provides a method for producing an L-amino acid, comprising culturing the microorganism in a medium.

In one specific embodiment, the method may further comprise recovering an L-amino acid from the cultured microorganism, a culture of the microorganism, a fermentation product of the microorganism, or the culture medium.

In another specific embodiment, the method may be a method for producing L-threonine.

Still another aspect of the present disclosure provides a composition for producing an L-amino acid comprising at least one selected from the variant polypeptide; a polynucleotide encoding the variant polypeptide; a vector comprising the same; a microorganism comprising the vector; a culture of the microorganism; and a fermentation product of the microorganism.

In one specific embodiment, the composition may be a composition for producing L-threonine.

### [Advantageous Effects]

Culturing a microorganism comprising the variant polypeptide of the nicotinamide nucleotide transhydrogenase α-subunit of the present disclosure enables the production of L-amino acids at a higher yield as compared to existing microorganisms having an unmodified polypeptide.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below. Additionally, a number of papers and patent documents have been cited throughout the present specification. The disclosures of the cited papers and patent documents are incorporated herein by reference in their entirety to describe the level of the technical field to which the present disclosure belongs and the contents of the present disclosure more clearly.

One aspect of the present disclosure provides a variant polypeptide of a nicotinamide nucleotide transhydrogenase α-subunit, wherein an amino acid corresponding to position 437 of SEQ ID NO: 10 is substituted with a different amino acid.

As used herein, the term "nicotinamide nucleotide transhydrogenase" refers to an enzyme that maintains the redox balance of a cell through the reaction [H⁺ + NADP⁺ + NADH ↔ H⁺ + NADPH + NAD⁺], which exists as a homodimer with each protomer comprising two soluble nucleotide binding domains that mediate hydride transfer between NAD(H) and NADP(H) and a proton-translocating transmembrane domain. In the present disclosure, the term may refer to a transmembrane pyridine nucleotide transhydrogenase that catalyzes the reduction of NADP⁺ to NADPH through the oxidation of NADH to NAD+, and is composed of two subunits, alpha and beta, encoded by the *pntA* and *pntB* genes, respectively.

The nicotinamide nucleotide transhydrogenase of the present disclosure may be used interchangeably with pntAB. Specifically, the nicotinamide nucleotide transhydrogenase of the present disclosure may be a protein having nicotinamide nucleotide transhydrogenase activity encoded by the *pntAB* (*pntA* and *pntB*) genes; however, the type is not particularly limited thereto as long as it exhibits an activity corresponding to that of nicotinamide nucleotide transhydrogenase.

The nicotinamide nucleotide transhydrogenase encoded by the *pntAB* gene is known in the art, and the amino acid and polynucleotide sequences of the nicotinamide nucleotide transhydrogenase can be obtained from publicly available databases (*e.g.*, GenBank), but are not limited thereto.

In one example, the nicotinamide nucleotide transhydrogenase may be composed of α-subunit PntA and β-subunit PntB.

The nicotinamide nucleotide transhydrogenase may comprise the amino acid sequence of SEQ ID NO: 10 or an amino acid sequence having at least 60% homology or identity thereto; and the amino acid sequence of SEQ ID NO: 11 or an amino acid sequence having at least 60% homology or identity thereto. However, it is not limited thereto as long as it has nicotinamide nucleotide transhydrogenase activity. Specifically, even if a protein comprises an amino acid sequence of SEQ ID NOs: 10 and 11 in which part of the sequence is deleted, modified, substituted, or added, the protein may fall within the scope of the present disclosure as long as the protein exhibits efficacy corresponding to that of a nicotinamide nucleotide transhydrogenase. Furthermore, the nicotinamide nucleotide transhydrogenase may have, comprise, consist of, or essentially consist of an amino acid sequence having at least 60%, 70%, 80%, 85%, 90%, 95%, 96%, 97%, 98%, or 99% homology or identity to the amino acid sequences of SEQ ID NOs: 10 and 11.

The protein subject to mutation introduction of the present disclosure may have nicotinamide nucleotide transhydrogenase activity. Specifically, it may comprise or be composed of the amino acid sequence of SEQ ID NO: 10, but is not limited thereto. An addition of meaningless sequences before or after the amino acid sequence of SEQ ID NO: 10, naturally occurring mutations, or silent mutations thereof are not excluded, and any protein having the same or corresponding activity as a protein comprising the amino acid sequence of SEQ ID NO: 10 may constitute a protein subject to mutation introduction of the present disclosure. For example, the protein subject to mutation introduction of the present disclosure may be a protein composed of an amino acid sequence having at least 60%, 70%, 75%, 80%, 85%, 90%, 95%, 96%, 97%, 98%, 99%, 99.5%, 99.7%, or 99.9% homology or identity to the amino acid sequence of SEQ ID NO: 10. Additionally, it is apparent that any polypeptide having an amino acid sequence, in which part of the sequence is deleted, modified, substituted, conservatively substituted, or added, also falls within the scope of the polypeptide subject to mutation introduction of the present disclosure, as long as the amino acid sequence has such a homology or identity and exhibits an equivalent efficacy to the polypeptide of the nicotinamide nucleotide transhydrogenase α-subunit.

As used herein, the terms "modified polypeptide", "modified protein" or "variant" refers to a polypeptide having at least one amino acid different from the amino acid sequence of the variant before mutation by conservative substitution and/or modification such that the functions and properties of the protein are retained. Such a variant may generally be identified by modifying at least one amino acid in the amino acid sequence of the polypeptide and evaluating the properties of the modified polypeptide. That is, the ability of the variant may be enhanced, unchanged, or reduced relative to a polypeptide before mutation. Additionally, some variants may include variants in which at least one portion, such as an N-terminal leader sequence or transmembrane domain, have been removed. Other variants may include variants in which a region has been partially removed from the N- and/or C-terminal of a mature protein. The term "variant" may be used interchangeably with terms such as variant, modified polypeptide, modified protein, variant polypeptide, variant protein, mutant, mutein, *etc*., but is not limited thereto, as long as the terms are used to indicate mutation.

Additionally, the variant may also comprise deletion or addition of amino acids that have minimal influence on the properties and secondary structure of a polypeptide. For example, a polypeptide may be conjugated with a signal (or leader) sequence at the N-terminus of a protein involved in the translocation of proteins co-translationally or post-translationally. Further, the polypeptide may also be conjugated with another sequence or linker to allow for identification, purification, or synthesis of the polypeptide.

The variant polypeptide of the present disclosure may be one in which the amino acid corresponding to position 437 from the N-terminus of SEQ ID NO: 10 is substituted with a different amino acid. The "variant polypeptide" may be referred to as "variant polypeptide of nicotinamide nucleotide transhydrogenase α-subunit", "nicotinamide nucleotide transhydrogenase α-subunit variant", "variant pntA", "pntA variant", *etc*. The nicotinamide nucleotide transhydrogenase α-subunit polypeptide subject to mutation introduction of the present disclosure may be used interchangeably with "PntA", and while not particularly limited, may be encoded by the *pntA* gene, and may be encoded by the *pntA* gene derived from *Corynebacterium variabile*, but is not limited thereto.

In the present disclosure, the amino acid before modification corresponding to position 437 of SEQ ID NO: 10 in the amino acid sequence before modification of the nicotinamide nucleotide transhydrogenase α-subunit subject to mutation introduction, i.e., the sequence that may serve as the parent sequence, may be valine (V), but is not limited thereto.

The variant polypeptide of a nicotinamide nucleotide transhydrogenase α-subunit of the present disclosure may be one in which an amino acid corresponding to position 437 of the amino acid sequence of SEQ ID NO: 10 is substituted with a different amino acid.

In one specific embodiment, the variant polypeptide may be one in which the amino acid corresponding to position 437 of the amino acid sequence of SEQ ID NO: 10 is substituted with an amino acid other than valine, which is the amino acid before substitution.

In another specific embodiment, the variant polypeptide may be one in which the amino acid corresponding to position 437 of SEQ ID NO: 10 is substituted with an amino acid selected from the group consisting of asparagine, serine, glycine, leucine, arginine, alanine, methionine, threonine, glutamine, proline, isoleucine, tryptophan, phenylalanine, histidine, cysteine, tyrosine, lysine, aspartate, and glutamic acid.

In any one embodiment of the foregoing embodiments, the variant polypeptide provided by the present disclosure may be one in which the amino acid corresponding to position 437 from the N-terminus of SEQ ID NO: 10 is substituted with alanine.

In another embodiment of the foregoing embodiments, the variant polypeptide provided by the present disclosure may comprise an amino acid sequence having at least 60%, 70%, 75%, 80%, 85%, 90%, 95%, 96%, 97%, 98%, 99%, 99.5%, or 99.8%, or less than 100% homology or identity to SEQ ID NO: 10.

For example, the variant polypeptide of the present disclosure may comprise an amino acid sequence in which the amino acid corresponding to position 437 of the amino acid sequence of SEQ ID NO: 10 is fixed to alanine, and which has at least 60%, 70%, 75%, 80%, 85%, 90%, 95%, 96%, 97%, 98%, 99%, 99.5%, or 99.8% homology or identity to SEQ ID NO: 10. Additionally, it is apparent that any variant polypeptide having an amino acid sequence, in which part of the sequence is deleted, modified, substituted, conservatively substituted, or added, also falls within the scope of the present disclosure, as long as the amino acid sequence has such a homology or identity and exhibits an equivalent efficacy to the variant polypeptide of the present disclosure.

Meanwhile, those skilled in the art would be able to identify the amino acid corresponding to position 437 of the amino acid sequence of SEQ ID NO: 10 in any amino acid sequence through sequence alignment known in the art. Further, even if not explicitly stated, it is apparent that the expression "amino acid at a specific position of a specific SEQ ID NO" in the present disclosure includes the "amino acid at the corresponding position" in any amino acid sequence.

In another specific embodiment, the variant polypeptide may consist of the amino acid sequence of SEQ ID NO: 14.

Specifically, the variant polypeptide of the present disclosure may have, comprise, consist of, or essentially consist of an amino acid sequence of SEQ ID NO:14, or an amino acid sequence having at least 60%, 70%, 80%, 85%, 90%, 95%, 96%, 97%, 98%, or 99% homology or identity to the amino acid sequences of SEQ ID NO: 14.

Furthermore, variant polypeptides having sequence additions or deletions that do not alter the function of the variant polypeptide, naturally occurring mutations, silent mutations, or conservative substitutions at the N-terminus, C-terminus, and/or within the amino acid sequence of the variant polypeptide of the present disclosure, may also be included within the scope of the variant polypeptide of the present disclosure.

The "another amino acid" is not limited as long as the amino acid differs from the amino acid before substitution. Meanwhile, when the present disclosure uses the expression "specific amino acid is substituted", it is apparent that the substitution is made with an amino acid different from the amino acid before substitution, even if it is not separately indicated that the substitution is made with a different amino acid.

Amino acids can generally be classified based on similarity in polarity, charge, solubility, hydrophobicity, hydrophilicity, and/or amphipathic nature of the residues.

For example, positively charged (basic) amino acids comprise arginine, lysine, and histidine; negatively charged (acidic) amino acids comprise glutamate and aspartate; amino acids with nonpolar side chains (nonpolar amino acids) comprise glycine, alanine, valine, leucine, isoleucine, methionine, phenylalanine, tryptophan, and proline; and amino acids with polar or hydrophilic side chains (polar amino acids) comprise serine, threonine, cysteine, tyrosine, asparagine, and glutamine. In another example, amino acids with electrically charged side chains (electrically charged amino acids) comprise arginine, lysine, histidine, glutamate, and aspartate, and amino acids with electrically uncharged side chains (also referred to as uncharged amino acids or neutral amino acids) comprise glycine, alanine, valine, leucine, isoleucine, methionine, phenylalanine, tryptophan, proline, serine, threonine, cysteine, tyrosine, asparagine, and glutamine. In another example, phenylalanine, tryptophan, and tyrosine may be categorized into aromatic amino acids. In another example, valine, leucine, and isoleucine may be categorized into branched amino acids. In another example, 20 types of amino acids may be classified into five groups by size, starting from the group of amino acids with relatively small volumes: glycine, alanine, and serine; cysteine, proline, threonine, aspartate, and asparagine; valine, histidine, glutamate, and glutamine; isoleucine, leucine, methionine, lysine, and arginine; and phenylalanine, tryptophan, and tyrosine. However, the classification of amino acids is not limited thereto.

For example, the expression that the "amino acid corresponding to position 437 of SEQ ID NO: 10 is substituted with a different amino acid" may refer to substitution with asparagine, serine, glycine, alanine, glutamate, phenylalanine, arginine, aspartate, cysteine, glutamine, histidine, proline, isoleucine, tyrosine, lysine, tryptophan, methionine, threonine, or leucine, excluding valine.

Even if the present disclosure describes a "protein having an amino acid sequence set forth in a specific SEQ ID NO", it is apparent that a protein having an amino acid sequence in which the sequence has been partially deleted, modified, substituted, conservatively substituted, or added may also be used in the present disclosure, as long as it has an activity identical or corresponding to that of the protein consisting of the amino acid sequence of the corresponding SEQ ID NO. For example, when exhibiting identical or equivalent activity to the variant protein, it is obvious that a protein with sequence additions, naturally-occurring mutations, or silent mutations or conservative substitutions thereof that do not alter the protein function is not excluded, and that such proteins having sequence additions or mutations fall within the scope of the present disclosure.

The term "position N" of the present disclosure may include position N and an amino acid position corresponding to position N. Specifically, the term may include the amino acid position corresponding to an arbitrary amino acid residue in a mature polypeptide of a specific amino acid sequence. The specific amino acid sequence may be the amino acid sequence of SEQ ID NO: 10.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the term "corresponding region" generally refers to a similar or corresponding position in a related protein or reference protein.

For example, based on the alignment of any amino acid sequence with SEQ ID NO: 10, each amino acid residue in the amino acid sequence may be numbered with reference to the position number of the amino acid residue corresponding to the amino acid residue of SEQ ID NO: 10. For example, a sequence alignment algorithm as described herein can identify the position of an amino acid or a position where modification such as substitution, insertion, or deletion occurs compared to a query sequence (also referred to as the "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16: 276-277), *etc*. may be used, but are not limited thereto, and sequence alignment programs, pairwise sequence comparison algorithms, *etc*., known in the art may be appropriately used.

As used herein, the term "conservative substitution" refers to the substitution of an amino acid with another amino acid having similar structural and/or chemical properties. Such an amino acid substitution may generally occur based on similarity in the polarity, charge, solubility, hydrophobicity, hydrophilicity, and/or amphipathic nature of residues. For example, positively charged (basic) amino acids include arginine, lysine, and histidine; negatively charged (acidic) amino acids include glutamate and aspartate; aromatic amino acids include phenylalanine, tryptophan, and tyrosine; and hydrophobic amino acids include alanine, valine, isoleucine, leucine, methionine, phenylalanine, tyrosine, and tryptophan. Furthermore, amino acids may be classified into those having electrically charged side chains and those having uncharged side chains. Amino acids having electrically charged side chains include aspartic acid, glutamic acid, lysine, arginine, and histidine; and amino acids having uncharged side chains may be further classified into nonpolar amino acids or polar amino acids, wherein nonpolar amino acids include glycine, alanine, valine, leucine, isoleucine, methionine, phenylalanine, tryptophan, and proline; and polar amino acids include serine, threonine, cysteine, tyrosine, asparagine, and glutamine. Typically, conservative substitution has little or no effect on the activity of the produced polypeptide. Typically, conservative substitution may have little or no effect on the activity of a protein or polypeptide.

Another aspect of the present disclosure provides a nicotinamide nucleotide transhydrogenase comprising the variant polypeptide.

The nicotinamide nucleotide transhydrogenase may comprise the variant polypeptide PntA and PntB.

That is, the nicotinamide nucleotide transhydrogenase of the present disclosure may be composed of the variant polypeptide of a nicotinamide nucleotide transhydrogenase α-subunit of the present disclosure and a nicotinamide nucleotide transhydrogenase β-subunit.

Still another aspect of the present disclosure provides a polynucleotide encoding the variant polypeptide.

As used herein, the term "polynucleotide" refers to a DNA or RNA strand having at least a certain length, which is a polymer of nucleotides in which nucleotide monomers are connected in a long chain by covalent bonds. More specifically, it refers to a polynucleotide fragment encoding the variant polypeptide.

The polynucleotide encoding the variant polypeptide of a nicotinamide nucleotide transhydrogenase α-subunit of the present disclosure may include any polynucleotide sequence as long as it is capable of forming nicotinamide nucleotide transhydrogenase together with β-subunit.

For example, the polynucleotide encoding the variant polypeptide of a nicotinamide nucleotide transhydrogenase α-subunit of the present disclosure may be a polynucleotide sequence encoding an amino acid sequence in which the amino acid corresponding to position 437 of SEQ ID NO: 10 is substituted with alanine, but is not limited thereto.

For example, the polynucleotide of the present disclosure may comprise a nucleotide sequence encoding the amino acid sequence of SEQ ID NO: 14. In addition, for example, the polynucleotide of the present disclosure may have or comprise the sequence of SEQ ID NO: 15. Furthermore, the polynucleotide of the present disclosure may consist of or essentially consist of the sequence of SEQ ID NO: 15.

The polynucleotide of the present disclosure may have various modifications in coding regions within a range that does not change the amino acid sequence of the variant polypeptide of the present disclosure, in consideration of degeneracy of codons or codons preferred in an organism in which the variant polypeptide of the present disclosure is to be expressed. Accordingly, it is apparent that a polynucleotide which can be translated, by codon degeneracy, into a polypeptide consisting of the amino acid sequence of the variant polypeptide of the present disclosure or a polypeptide having a homology or identity thereto may also be included in the present disclosure. For example, the polynucleotide of the present disclosure may consist of or comprise the sequence of SEQ ID NO: 15, or a degenerate sequence thereof.

For example, the polynucleotide of the present disclosure may include a nucleotide sequence having at least 60%, 70%, 75%, 80%, 85%, 90%, 95%, 96%, 97%, 98%, 99%, 99.5%, 99.7%, or 99.8% homology or identity to the sequence of SEQ ID NO: 12, in which the codon corresponding to the codon at positions 1309 to 1311 of SEQ ID NO: 12 is substituted with a codon encoding an amino acid different from the original amino acid, for example, alanine, but is not limited thereto. Additionally, it is apparent that any variant having a polynucleotide sequence, in which the sequence is partially deleted, modified, substituted, conservatively substituted, or added, also falls within the scope of the present disclosure, as long as the polynucleotide sequence has such a homology or identity and encodes the amino acid sequence of the variant polypeptide of a nicotinamide nucleotide transhydrogenase α-subunit of the present disclosure.

In another example, the polynucleotide of the present disclosure may have or comprise a nucleotide sequence having at least 60%, at least 70%, at least 75%, at least 80%, at least 85%, at least 90%, at least 95%, at least 96%, at least 97%, at least 98%, and less than 100% homology or identity to the sequence of SEQ ID NO: 15, or may consist of, essentially consist of, or comprise a nucleotide sequence having at least 60%, at least 70%, at least 75%, at least 80%, at least 85%, at least 90%, at least 95%, at least 96%, at least 97%, at least 98%, and less than 100% homology or identity to the sequence of SEQ ID NO: 15.

In addition, the polynucleotide of the present disclosure may comprise a probe that can be prepared from a known gene sequence without limitation, for example, any sequence capable of hybridizing with a complementary sequence to all or a part of the polynucleotide sequence of the present disclosure under stringent conditions.

The term "stringent conditions" refers to conditions that enable specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having a high homology or identity of at least 60%, at least 70%, at least 75%, at least 80%, at least 85%, at least 90%, at least 95%, at least 96%, at least 97%, or at least 99% are hybridized with each other and polynucleotides having a homology or identity lower than the above are not hybridized with each other; or common washing conditions of Southern hybridization, that is, washing once, specifically, twice or three times at a salt concentration and a temperature corresponding to 60°C, 1 × SSC, 0.1% SDS, specifically, 60°C, 0.1 × SSC, 0.1% SDS, and more specifically 68°C, 0.1 × SSC, 0.1% SDS.

Hybridization requires that two nucleic acids have complementary sequences, although mismatches between nucleotides may be possible depending on the stringency of hybridization. The term "complementary" is used to describe the relationship between nucleotide bases capable of hybridizing with each other. For example, regarding DNA, adenine is complementary to thymine, and cytosine is complementary to guanine. Therefore, the polynucleotide of the present disclosure may also include an isolated nucleic acid fragment complementary to the entire sequence as well as a nucleic sequence substantially similar thereto.

Specifically, polynucleotides having homology or identity to the polynucleotide of the present disclosure may be detected using hybridization conditions comprising a hybridization step at a Tₘ value of 55 °C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (*e.g.*, Sambrook *et al*., supra).

As used herein, the term "homology" or "identity" refers to a degree of similarity between two given amino acid sequences or nucleotide sequences, and may be expressed as a percentage. The terms homology and identity may often be used interchangeably.

The sequence homology or identity of conserved polynucleotides or polypeptides may be determined by a standard alignment algorithm, and default gap penalties established by the program used may be applied together. Substantially, homologous or identical sequences are generally capable of hybridizing with the whole sequence or a part of the sequence under moderately or highly stringent conditions. It is apparent that hybridization also includes hybridization with a polynucleotide containing a general codon or a codon considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al., Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ET AL.] (1988) SIAM J Applied Math 48: 1073)). For example, homology, similarity, or identity may be determined using BLAST of the National Center for Biotechnology Information, or ClustalW.

The homology, similarity, or identity between polynucleotides or polypeptides may be determined by, for example, comparing sequence information using a GAP computer program, such as Needleman et al., (1970), J Mol Biol. 48:443, as described in, for example, Smith and Waterman, Adv. Appl. Math (1981) 2:482. Briefly, a GAP program may be defined as the value acquired by dividing the number of similarly aligned symbols (namely, nucleotides or amino acids) by the total number of symbols in the shorter of two sequences. The default parameters for the GAP program may comprise: (1) a binary comparison matrix (comprising values of 1 for identity and 0 for non-identity) and a weighted comparison matrix of Gribskov et al. (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps. Accordingly, the terms "homology" or "identity" as used herein indicate the relevance between sequences.

Still another aspect of the present disclosure provides a vector comprising the polynucleotide of the present disclosure. The vector may be an expression vector for expressing the polynucleotide in a microorganism, but is not limited thereto.

As used herein, the term "vector" of the present disclosure may comprise a DNA construct comprising the nucleotide sequence of a polynucleotide encoding the target polypeptide operably linked to a suitable expression regulatory region (or expression regulatory sequence) so as to enable expression of the target polypeptide in a suitable host cell. The expression regulatory region may comprise a promoter capable of initiating transcription, any operator sequence for controlling such transcription, a sequence encoding a suitable mRNA ribosome binding site, and a sequence controlling termination of transcription and translation. The vector, after transformation into a suitable microorganism, may be replicated or function independently of the genome of the host, or may be integrated into the genome itself.

The vector used in the present disclosure is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, and Charon21A, *etc*. may be used; and as a plasmid vector, those based on pDZ, pBR, pUC, pBluescriptII, pGEM, pTZ, pCL, pET, *etc*. may be used. Specifically, vectors such as pDZ, pDC, pDCM2, pDC24, pACYC177, pACYC184, pCL, pECCG117, pUC19, pBR322, pMW118, and pCC1BAC may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. A selection marker for confirming the chromosomal insertion may be further included. The selection marker is used to screen cells transformed with the vector, i.e., to confirm the insertion of the target nucleic acid molecule, and markers that confer selectable phenotypes, such as drug resistance, auxotrophy, resistance to cytotoxic agents, or expression of surface polypeptides, may be used. In an environment treated with a selective agent, only cells expressing the selection marker survive or exhibit a different phenotypic trait, thereby enabling selection of transformed cells.

As used herein, the term "transformation" refers to the introduction of a vector comprising a polynucleotide encoding a target polypeptide into a microorganism such that the polypeptide encoded by the polynucleotide can be expressed in the microorganism. As long as the transformed polynucleotide can be expressed within a microorganism, the transformed polynucleotide may be located within the chromosome of the microorganism or exist extrachromosomally. Further, the polynucleotide comprises DNA and/or RNA encoding the target polypeptide. The polynucleotide may be introduced in any form as long as the polynucleotide can be introduced and expressed in a microorganism. For example, the polynucleotide may be introduced into a microorganism in the form of an expression cassette, which is a gene construct including all factors required for self-expression. The expression cassette may usually comprise a promoter operably linked to the polynucleotide, a transcription termination signal, a ribosome binding site, and a translation termination signal. The expression cassette may be in the form of a self-replicating expression vector. Further, the polynucleotide may be introduced into the microorganism as-is and operably linked to a sequence required for expression in the microorganism, but is not limited thereto.

Further, as used herein, the term "operably linked" means that the polynucleotide sequence is functionally linked to a promoter sequence that initiates and mediates transcription of the polynucleotide encoding the target variant polypeptide of the present disclosure.

The method for transforming the vector of the present disclosure includes any method of introducing a nucleic acid into a cell and may be performed by selecting a suitable standard technique as known in the art depending on the host cell. Examples include electroporation, calcium phosphate (CaPO₄) precipitation, calcium chloride (CaCl₂) precipitation, microinjection, polyethylene glycol (PEG) method, DEAE-dextran method, cationic liposome method, and lithium acetate-DMSO method, but are not limited thereto.

Still another aspect of the present disclosure provides a microorganism comprising at least one selected from the variant polypeptide; a polynucleotide encoding the variant polypeptide; and a vector comprising the same.

Still another aspect of the present disclosure provides a microorganism further comprising at least one selected from a nicotinamide nucleotide transhydrogenase β-subunit; a polynucleotide encoding the β-subunit; and a vector comprising the same.

In one specific embodiment, the microorganism of the present disclosure may be a microorganism having an ability to produce an L-amino acid or a derivative thereof.

As used herein, the term "microorganism (or strain)" comprises all wild-type microorganisms or naturally or artificially genetically modified microorganisms, may be a microorganism in which a particular mechanism is weakened or enhanced due to insertion of a foreign gene, enhancement or inactivation of the activity of an endogenous gene, *etc*., and may be a microorganism comprising genetic modification to produce a target polypeptide, protein, or product. In the present disclosure, the terms "microorganism", "strain", and "microorganism" have the same meaning and may be used interchangeably without limitation.

As used herein, the term "recombinant microorganism" refers to a microorganism that has been genetically modified to exhibit a different genotype and/or phenotype compared to a naturally occurring microorganism (*e.g.*, when genetic modification affects how the nucleic acid sequence is encoded in the microorganism), and may comprise all progeny or potential progeny of the microorganism. In the present disclosure, the terms "recombinant microorganism", "genetically modified microorganism", "recombinant host cell", "recombinant cell", and "recombinant strain" may be used interchangeably. The recombinant microorganism may, for example, express a gene that is not found in its natural (non-recombinant) form, not express a gene that is expressed in its natural form, or express a natural gene in a manner different from that in which it is expressed in its natural form.

As used herein, the term "microorganism producing L-amino acid" refers to a prokaryotic or eukaryotic microbial strain capable of producing L-amino acids within a living organism, and may include both a microorganism wherein an L-amino acid-producing ability has been imparted into a parent strain lacking the L-amino acid-producing ability, and a microorganism that intrinsically has an L-amino acid-producing ability. The L-amino acid-producing ability may be imparted or enhanced by strain improvement.

In one embodiment, the microorganism of the present disclosure may be a microorganism that naturally has a variant polypeptide of a nicotinamide nucleotide transhydrogenase α-subunit or an L-amino acid-producing ability; a microorganism wherein a parent strain that does not have a variant polypeptide of a nicotinamide nucleotide transhydrogenase α-subunit or an L-amino acid-producing ability has been introduced with the variant polypeptide of the present disclosure or a polynucleotide encoding the same (or a vector comprising the polynucleotide); and/or a microorganism imparted with an L-amino acid-producing ability, but is not limited thereto.

In one embodiment, the microorganism of the present disclosure includes both a microorganism in which a chromosomal gene encoding a nicotinamide nucleotide transhydrogenase α-subunit polypeptide is mutated, and thereby comprises the variant polypeptide sequence of the nicotinamide nucleotide transhydrogenase α-subunit of the present disclosure; and/or a microorganism comprising the variant polypeptide of the nicotinamide nucleotide transhydrogenase α-subunit of the present disclosure, from introducing a polynucleotide encoding the variant polypeptide of the nicotinamide nucleotide transhydrogenase α-subunit of the present disclosure, or a vector comprising the same; but the microorganism is not limited thereto.

As used herein, the term "unmodified microorganism" does not exclude a strain comprising mutations that may occur naturally in a microorganism, and may refer to a wild-type strain or a natural strain *per se*, or a strain before a trait is altered by genetic variation due to natural or artificial factors. For example, the unmodified microorganism may refer to a strain into which the variant polypeptide of a nicotinamide nucleotide transhydrogenase α-subunit of the present disclosure has not been introduced, or a strain before introduction thereof. The term "unmodified microorganism" may be used interchangeably with "strain before modification", "microorganism before modification", "non-mutant strain", "unmodified strain", "non-mutant microorganism", or "reference microorganism".

The microorganism having L-amino acid-producing ability of the present disclosure may be, but is not limited to: a microorganism comprising at least one of the variant polypeptide of the present disclosure, the polynucleotide of the present disclosure, and a vector comprising the polynucleotide of the present disclosure; a microorganism modified to express the variant polypeptide of the present disclosure or the polynucleotide of the present disclosure; a microorganism expressing the variant polypeptide of the present disclosure or the polynucleotide of the present disclosure (*e.g.*, a recombinant strain); or a microorganism having an activity of the variant polypeptide of the present disclosure (*e.g.*, a recombinant strain).

In one example, the microorganism of the present disclosure is a cell or microorganism that is transformed with a polynucleotide encoding the variant polypeptide of the present disclosure or a vector comprising the same, and expresses the variant polypeptide of the present disclosure. The strain of the present disclosure may include all microorganisms comprising the variant polypeptide of the present disclosure and capable of producing an L-amino acid or derivatives thereof. For example, the microorganism of the present disclosure may be a recombinant strain in which a polynucleotide encoding the variant polypeptide of the present disclosure is introduced into a naturally occurring wild-type microorganism or a microorganism having an L-amino acid-producing ability - such that the variant polypeptide of a nicotinamide nucleotide transhydrogenase α-subunit is expressed or a variant nicotinamide nucleotide transhydrogenase comprising the variant polypeptide of the present disclosure is expressed - resulting in increased L-amino acid-producing ability. The recombinant strain with increased L-amino acid-producing ability may be a microorganism having increased L-amino acid-producing ability compared to a naturally occurring wild-type microorganism or an unmodified microorganism (*e.g.*, a microorganism expressing wild-type nicotinamide nucleotide transhydrogenase or a microorganism not expressing the variant polypeptide of the present disclosure), but is not limited thereto. In one example, the microorganism with an increased L-amino acid-producing ability of the present disclosure may be a microorganism with an increased L-amino acid-producing ability compared to a microorganism comprising a polypeptide of SEQ ID NO: 10 or a polynucleotide encoding the same, but is not limited thereto. In one example, the unmodified microorganism, which is the reference strain for comparing whether L-amino acid-producing ability is increased, may be the wild-type *Corynebacterium glutamicum* strain KCCM 12502P (CA09-0903), but is not limited thereto.

The microorganism of the present disclosure may include all microorganisms capable of expressing the variant polypeptide of a nicotinamide nucleotide transhydrogenase α-subunit of the present disclosure by various known methods, in addition to the introduction of the nucleic acid or vector.

In one example, the microorganism with increased L-amino acid-producing ability may have an increase of at least about 1%, specifically at least about 1%, at least about 2%, at least about 3%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19%, at least about 20%, at least about 25%, at least about 30%, at least about 35%, at least about 40%, or at least about 50% (the upper limit is not particularly limited, and may be, for example, about 200% or less, about 150% or less, about 100% or less, about 90% or less, about 80% or less, about 70% or less, about 60% or less, about 55% or less, or about 50% or less) compared to the L-amino acid-producing ability of the parent strain before mutation or the unmodified microorganism; however, it is not limited thereto, as long as the increase in producing ability is a positive value compared to the producing ability of the parent strain before mutation or the unmodified microorganism. In another example, the recombinant strain with increased L-amino acid-producing ability may have an L-amino acid-producing ability that is increased by at least about 1.01-fold, at least about 1.02-fold, at least about 1.03-fold, at least about 1.04-fold, at least 1.05-fold, at least 1.06-fold, at least 1.07-fold, at least 1.08-fold, at least 1.09-fold, at least about 1.1-fold, at least about 1.11-fold, at least about 1.12-fold, at least about 1.13-fold, at least about 1.14-fold, at least about 1.15-fold, at least about 1.16-fold, at least about 1.17-fold, at least about 1.18-fold, at least about 1.19-fold, at least about 1.20-fold, at least about 1.25-fold, at least about 1.30-fold, at least about 1.35-fold, at least about 1.40-fold, at least about 1.45-fold, or at least about 1.50-fold (the upper limit is not particularly limited, and may be, for example, about 10-fold or less, about 5-fold or less, about 3-fold or less, about 2-fold or less, or about 1.5-fold or less) compared to the parent strain before mutation or the unmodified microorganism; however, it is not limited thereto. As used herein, the term "about" refers to a range which includes all of ±0.5, ±0.4, ±0.3, ±0.2, ±0.1, *etc*., and includes all of the values that are equivalent or similar in range to the value that follows the term, but the range is not limited thereto.

According to any one of the preceding specific embodiments, the microorganism of the present disclosure may be a microorganism of the genus *Corynebacterium, Escherichia, Erwinia, Serratia, Providencia, Pseudomonas, Leptospira, Salmonella, Brevibacteria, Hyphomonas, Chromobacterium,* and *Nocardia,* or fungi or yeast, and specifically may be a microorganism belonging to the genus *Corynebacterium,* but is not limited thereto.

In one embodiment of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.* Specifically, the microorganism of the present disclosure may be a microorganism of the genus *Corynebacterium,* and more specifically *Corynebacterium glutamicum,* but is not limited thereto.

Meanwhile, the microorganism of the genus *Corynebacterium* having enhanced L-amino acid-producing ability of the present disclosure comprises any natural wild-type microorganism, microorganism of the genus *Corynebacterium* with increased L-amino acid-producing ability by enhancing or weakening the expression of a gene related to the L-amino acid production mechanism, or microorganism of the genus *Corynebacterium* with increased L-amino acid-producing ability by introducing or enhancing an exogenous gene. In one specific embodiment, the microorganism of the present disclosure may include a microorganism of the genus *Corynebacterium* having improved L-amino acid-producing ability through the introduction of the variant polypeptide of the present disclosure or a variant nicotinamide nucleotide transhydrogenase comprising the same, but is not limited thereto.

As used herein, an "increase" in protein (polypeptide) activity means that the activity of a protein (polypeptide) is increased compared to its endogenous activity in a host cell (microorganism). The increase may be used interchangeably with terms such as "activation", "up-regulation", "overexpression", or "enhancement". The host cell (microorganism) may be a prokaryotic or eukaryotic microorganism.

The increase in protein (polypeptide) activity may comprise both the case in which a host cell (microorganism) exhibits a protein (polypeptide) activity that it did not inherently possess, and the case in which a host cell exhibits an improved protein (polypeptide) activity compared to its endogenous activity or its activity before modification.

For example, the case in which it "exhibits a protein (polypeptide) activity that it did not inherently possess" or the case in which it exhibits an improved protein (polypeptide) activity may result from "introduction of a protein (polypeptide)", but is not limited thereto.

As used herein, the term "introduction" of a protein (polypeptide) refers to a case in which a gene that a microorganism did not originally possess is expressed within the microorganism so that the activity of a specific protein is exhibited, or to a case in which the polypeptide activity is strengthened, increased, or improved compared to its endogenous activity or its activity before modification. For example, it may result from the introduction of a gene encoding the protein (polypeptide) into a host cell (microorganism). For example, a polynucleotide encoding a specific protein (polypeptide) may be introduced into a chromosome of a host cell (microorganism), or a vector comprising the polynucleotide encoding a specific protein (polypeptide) may be introduced into a host cell (microorganism), thereby exhibiting or enhancing the activity thereof.

The "endogenous activity" refers to the activity of a specific protein (polypeptide) originally possessed by a host cell (microorganism) before transformation or an unmodified host cell (microorganism), when a trait is altered by genetic modification due to natural or artificial factors. This term may be used interchangeably with "activity before modification".

An increase in activity of a protein (polypeptide) compared to its endogenous activity means that the activity and/or concentration (expression level) of the protein (polypeptide) in a host cell (microorganism) is enhanced compared to the activity and/or concentration (expression level) of the protein (polypeptide) originally possessed by the host cell (microorganism) before modification or an unmodified host cell (microorganism).

In one example, the increase may refer to the emergence of activity of the corresponding protein (polypeptide) that was absent, or the increase in activity or concentration of the protein, relative to the activity or concentration in the host cell (microorganism) before transformation or in an unmodified host cell (microorganism), typically by at least about 1%, at least about 10%, at least about 25%, at least about 50%, at least about 75%, at least about 100%, at least about 150%, at least about 200%, at least about 300%, at least about 400%, or at least about 500%, up to at least about 1000% or at least about 2000% or more, but is not limited thereto.

The increase in activity of a protein (polypeptide) may be achieved by introducing a foreign protein (polypeptide) or by increasing the activity of an endogenous protein (polypeptide). Whether the activity of a protein (polypeptide) has increased may be confirmed from the increase in the activity level of the protein (polypeptide), its expression level, or the amount of product resulting from the activity of the corresponding protein (polypeptide).

The increase in the activity of a protein (polypeptide) can be achieved by various methods well known in the art, and is not limited as long as the activity of the desired protein (polypeptide) can be increased compared to that of the host cell (microorganism) before modification. Specifically, genetic engineering and/or protein engineering well known to those skilled in the art as common methods in molecular biology may be used, but the method is not limited thereto (*e.g*., Sitnicka et al. Functional Analysis of Genes. Advances in Cell Biology. 2010, Vol. 2. 1-16, Sambrook et al. Molecular Cloning 2012, *etc*.).

Specifically, the increase in the activity of a protein (polypeptide) of the present disclosure may result from:
1) increase in the intracellular copy number of a polynucleotide encoding the protein (polypeptide);
2) modification of a gene expression regulatory region on a chromosome encoding the protein (polypeptide) (*e.g*., introduction of a modification on the expression regulatory region, replacement with a sequence exhibiting stronger activity, or insertion of a sequence exhibiting stronger activity);
3) modification of a base sequence encoding a start codon or the 5'-UTR of a gene transcript encoding the protein (polypeptide);
4) modification of the amino acid sequence of the protein (polypeptide) to increase the activity of the protein (polypeptide);
5) modification of a polynucleotide sequence encoding the protein (polypeptide) to increase the activity of the protein (polypeptide) (*e.g*., modification of a polynucleotide sequence of a gene encoding the protein (polypeptide), such that the gene encodes a protein (polypeptide) modified to have increased activity);
6) introduction of a foreign protein (polypeptide) exhibiting the activity of the protein (polypeptide), or a foreign polynucleotide encoding the same;
7) codon optimization of a polynucleotide encoding the protein (polypeptide);
8) selection and modification or chemical modification of exposed regions of the protein (polypeptide) through analysis of the tertiary structure thereof;
9) regulation of the cellular localization of the protein (polypeptide); or
10) a combination of two or more selected from 1) to 9) above, but is not particularly limited thereto.

For example,
The 1) increase in the intracellular copy number of a polynucleotide encoding the protein (polypeptide) may be achieved by introducing a vector comprising the polynucleotide encoding the protein (polypeptide) operably linked to a suitable regulatory sequence into a host cell (microorganism). Alternatively, it may be achieved by introducing one copy, or two or more copies, of a polynucleotide encoding the protein (polypeptide) operably linked to a suitable regulatory sequence into a chromosome of a host cell (microorganism). The introduction into a chromosome may be performed by introducing a vector capable of inserting the polynucleotide into a chromosome of a host cell (microorganism) into the host cell (microorganism), but is not limited thereto. The vector is as described above. With respect to the polynucleotide sequence encoding the protein, the regulatory sequence may be a native sequence (of the same origin) or a foreign sequence (derived from a different gene), a variant thereof, or another artificial sequence, and may induce the expression of the polynucleotide in a host cell (microorganism).

The 2) replacement of the expression regulatory region (or expression regulatory sequence) of a gene encoding the protein (polypeptide) on the chromosome with a sequence having strong activity may be achieved, for example, by introducing a modification into the sequence through deletion, insertion, substitution, or a combination thereof to further increase the activity of the expression regulatory region, or by replacing the sequence with a sequence having stronger activity. The expression regulatory region may comprise, but is not particularly limited to, a promoter, an operator sequence, a sequence encoding a ribosome-binding site, a sequence regulating the termination of transcription and translation, and the like. In one example, it may be replacing the original promoter with a strong promoter, but is not limited thereto.

Examples of known strong promoters include cj1 to cj7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but the strong promoter is not limited thereto.

The 3) modification of a base sequence encoding a start codon or the 5'-UTR of a gene transcript encoding the protein (polypeptide) may be achieved, for example, by modifying the base sequence such that it encodes a different initiation codon with a higher expression rate of the protein (polypeptide) compared to the endogenous initiation codon, or by modifying the base sequence such that it encodes a ribosome binding site (RBS) sequence with a higher expression rate of the protein (polypeptide) compared to the endogenous RBS sequence, but is not limited thereto.

The 4) and 5) modification of the amino acid sequence or polynucleotide sequence of the protein (polypeptide) may be achieved by introducing a modification on the sequence of the amino acid sequence of the protein (polypeptide) or the polynucleotide sequence encoding the protein (polypeptide) through deletion, insertion, substitution, or a combination thereof to enhance the activity of the protein (polypeptide), or by replacing the sequence with an amino acid sequence or polynucleotide sequence modified to have increased activity, but are not limited thereto. The replacement may, for example, be performed by inserting the polynucleotide into a chromosome by homologous recombination, but is not limited thereto.

The 6) introduction of a foreign polynucleotide exhibiting the activity of the protein (polypeptide) may be achieved by introducing into a host cell (microorganism) a foreign polynucleotide encoding a protein (polypeptide) that exhibits the same/similar activity to that of the protein (polypeptide). The foreign polynucleotide is not limited by its origin or sequence, as long as it exhibits the same/similar activity to that of the protein (polypeptide). The introduction may be performed by a transformation method known in the art appropriately selected by those skilled in the art, and the expression of the introduced polynucleotide in the host cell enables the production of the protein (polypeptide), thereby increasing its activity.

The 7) codon optimization of a polynucleotide encoding the protein (polypeptide) may be achieved by codon-optimizing an endogenous polynucleotide to increase the transcription or translation within a host cell (microorganism), or by optimizing the codons of a foreign polynucleotide such that optimized transcription and translation thereof may be achieved within the host cell.

The 8) selection and modification or chemical modification of exposed regions of the protein (polypeptide) through analysis of the tertiary structure thereof may be achieved, for example, by comparing the sequence information of the protein (polypeptide) to be analyzed with a database storing the sequence information of known proteins to identify template protein candidates according to the degree of sequence similarity and confirming the structure based on the information, thereby selecting the exposed site to be modified or chemically modified and transforming or modifying the same.

The 9) regulation of the cellular localization of the protein (polypeptide) may be achieved by targeting the protein (polypeptide) to a particular intracellular organelle or a particular intracellular space. For example, it may be achieved by targeting the protein (polypeptide) to the periplasm or cytoplasm through the addition or removal of a leader sequence that functions in the targeting of proteins (polypeptides), but is not limited thereto.

Such enhancement of the protein (polypeptide) activity may refer to an increase in the activity or concentration of the corresponding protein (polypeptide) relative to the activity or concentration of the protein (polypeptide) expressed in a wild-type strain or a host cell (microorganism) before modification, or an increase in the amount of product produced by the protein (polypeptide), but is not limited thereto.

The modification of a part or all of a polynucleotide in the microorganism of the present disclosure may be induced by (a) a method using homologous recombination with a vector for chromosomal insertion in the microorganism or genome editing using an engineered nuclease (*e.g*., CRISPR-Cas9), and/or (b) light, such as ultraviolet rays and radiation, *etc*. and/or chemical treatments, but is not limited thereto.

The microorganism of the present disclosure may be one in which an L-amino acid-producing ability has been enhanced.

In the present disclosure, "L-amino acid" includes all L-amino acids that can be produced by microorganisms through metabolic processes from various carbon sources, and may specifically include L-threonine, but is not limited thereto.

In one specific embodiment of the present disclosure, the microorganism of the genus *Corynebacterium* may have increased L-threonine-producing ability compared to an unmodified microorganism.

Still another aspect of the present disclosure provides a method for producing an L-amino acid, comprising culturing, in a medium, a microorganism comprising at least one selected from the variant polypeptide of the present disclosure; a polynucleotide encoding the variant polypeptide; and a vector comprising the same.

The microorganism is as described in another aspect.

As used herein, the term "culturing" refers to growing the microorganism of the present disclosure under appropriately controlled environmental conditions. In the present disclosure, the culturing process may be performed in a suitable medium known in the art under suitable culturing conditions known in the art. Such a culturing process may be readily adjusted and used by those skilled in the art according to the selected strain. Specifically, the culturing may be batch culturing, continuous culturing, and/or fed-batch culturing, but is not limited thereto.

The microorganism of the present disclosure may be cultured in a common medium containing appropriate carbon sources, nitrogen sources, phosphorus sources, inorganic compounds, amino acids and/or vitamins, *etc*., while controlling the temperature, pH, *etc*. under aerobic conditions.

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (i.e., molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monopotassium phosphate, dipotassium phosphate, or sodium-containing salts corresponding thereto may be used. As for the inorganic compound, sodium chloride, calcium chloride, iron chloride, magnesium sulfate, iron sulfate, manganese sulfate, calcium carbonate, *etc*. may be used. Additionally, amino acids, vitamins, and/or appropriate precursors, *etc*. may be included. These components or precursors may be added to the medium in a batchwise or continuous manner. However, the medium is not limited thereto.

During the cultivation of the microorganism of the present disclosure, the pH of the medium may be adjusted by adding compounds such as ammonium hydroxide, potassium hydroxide, ammonia, phosphoric acid, sulfuric acid, *etc*. to the medium in a suitable manner. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas may be injected, or nitrogen, hydrogen, or carbon dioxide gas may be injected. However, the culturing conditions are not limited thereto.

Further, the culture medium may contain metal salts such as magnesium sulfate or iron sulfate necessary for growth. Lastly, in addition to the aforementioned substances, essential growth substances such as amino acids and vitamins may be used. Additionally, suitable precursors may be used in the culture medium. The aforementioned raw materials may be added to the culture in a batch or continuous manner by a suitable method during the cultivation process, but are not limited thereto.

During the cultivation of the microorganism of the present disclosure, the pH of the culture may be adjusted by adding compounds such as ammonium hydroxide, potassium hydroxide, ammonia, phosphoric acid, sulfuric acid, *etc*. to the culture in a suitable manner. During the culturing, an antifoaming agent such as fatty acid polyglycol ester may be used to suppress foaming. To maintain an aerobic state of the culture, oxygen or oxygen-containing gas may be injected into the culture. To maintain an anaerobic or microaerobic state of the medium, no gas may be injected, or nitrogen, hydrogen, or carbon dioxide gas may be injected. However, the culturing conditions are not limited thereto.

In the culturing of the present disclosure, the culturing temperature may be maintained at 20°C to 35°C, specifically 25°C to 35°C. The culturing may be continued until a desired amount of useful substance is obtained, and culturing may be performed for about 10 to 160 hours, about 20 to 130 hours, about 24 to 120 hours, about 36 to 120 hours, about 48 to 120 hours, about 48 hours or more, or about 48 hours, about 72 hours, or about 120 hours, but is not limited thereto.

The L-amino acid produced by the culturing of the present disclosure may be secreted into the medium or remain in the cells.

In one specific embodiment of the present disclosure, the L-amino acid may be L-threonine, but is not limited thereto.

In one specific embodiment, the method for producing an L-amino acid of the present disclosure may further comprise preparing the microorganism of the present disclosure, preparing a medium for culturing the microorganism, or a combination thereof (in any order), for example, prior to culturing.

The method for producing an L-amino acid of the present disclosure may further comprise recovering a target substance, specifically an L-amino acid or derivatives thereof, from the cultured microorganism, a culture of the microorganism, a fermentation product of the microorganism, or the culture medium. The recovery may be further comprised after the culturing.

The recovery may be collecting the target L-amino acid or derivatives thereof using a suitable method known in the art according to the culturing method of the microorganism of the present disclosure, for example, a batch, continuous, or fed-batch culturing method, *etc*. For example, centrifugation, filtration, treatment with a crystallized protein precipitating agent (salting-out), extraction, sonication, ultrafiltration, dialysis, various types of chromatography such as molecular sieve chromatography (gel filtration), adsorption chromatography, ion exchange chromatography, affinity chromatography, *etc*., HPLC, and a combination of these methods may be used, and the target substance, specifically L-amino acid, can be recovered from the medium or the microorganism using suitable methods known in the art.

Further, the method for producing an L-amino acid of the present disclosure may further comprise purification. The purification may be performed using a suitable method known in the art. In one example, when the method for producing an L-amino acid of the present disclosure comprises both recovery and purification, the recovery and purification may be performed continuously or non-continuously regardless of the order, or integrated into a simultaneous or a single step, but the method is not limited thereto.

In the method of the present disclosure, the variant polypeptide, introduction, L-amino acid or derivatives thereof, *etc*. are as described in other aspects above.

Still another aspect of the present disclosure provides a composition for producing an L-amino acid comprising at least one selected from the variant polypeptide of the present disclosure; a polynucleotide encoding the variant polypeptide; a vector comprising the same; a microorganism comprising the vector; a culture of the microorganism; and a fermentation product of the microorganism.

The composition of the present disclosure may further comprise any suitable excipient commonly used in compositions for producing amino acids, and such excipients may be, for example, preservatives, wetting agents, dispersing agents, suspending agents, buffers, stabilizers, isotonic agents, *etc*., but are not limited thereto.

In one specific embodiment, the composition of the present disclosure may comprise each component in a microbially effective amount or in an amount that can be suitably present in a composition for production.

In the composition of the present disclosure, the variant polypeptide, introduction, L-amino acid, *etc*. are as described in other aspects above.

Still another aspect of the present disclosure provides use of a microorganism having improved L-amino acid-producing ability, wherein at least one selected from the variant polypeptide of the present disclosure and a polynucleotide encoding the variant polypeptide has been introduced, for producing an L-amino acid or derivatives thereof.

In the use of the present disclosure, the variant polypeptide, introduction, L-amino acid, *etc*. are as described in other aspects above.

Another aspect of the present disclosure provides a method for preparing a microorganism having improved L-amino acid-producing ability, comprising introducing at least one selected from the variant polypeptide of the present disclosure and a polynucleotide encoding the variant polypeptide, into a microorganism belonging to the genus *Corynebacterium* having L-amino acid-producing ability.

Another aspect of the present disclosure provides a method for increasing L-amino acid-producing ability, comprising introducing at least one selected from the variant polypeptide of the present disclosure and a polynucleotide encoding the variant polypeptide, into a microorganism belonging to the genus *Corynebacterium* having L-amino acid-producing ability.

In the method of the present disclosure, the variant polypeptide, introduction, L-amino acid, *etc*. are as described in other aspects above.

### [Mode for Carrying Out the Invention]

Hereinafter, the present disclosure will be described in more detail by way of Examples and Experimental Examples. However, these Examples and Experimental Examples are given for illustrative purposes only, and the scope of the present disclosure is not intended to be limited by these Examples and Experimental Examples.

### Example 1: Construction of recombinant vector for introduction of foreign nicotinamide nucleotide transhydrogenase (pntAB)

### Example 1-1: Construction of plasmid for gene insertion

To insert a foreign nicotinamide nucleotide transhydrogenase (hereinafter referred to as "pntAB") gene into the chromosome of *Corynebacterium glutamicum,* NCgl1287, which is known as a gene encoding a transposon, was used as an insertion site (J Bacteriol. 2011 Mar; 193(5): 1237-1249). A vector for NCgl1287 deletion and target gene insertion was constructed in order to replace the NCgl1287 gene with foreign pntAB. To construct the vector, PCR was performed using the chromosome of *Corynebacterium glutamicum* ATCC13032 as a template with primer pairs of SEQ ID NO: 1 and SEQ ID NO: 2, and SEQ ID NO: 3 and SEQ ID NO: 4, respectively. The primer sequences used to perform each of the PCR reactions are as shown in Table 1 below.

Pfu Ultra^{™} high-fidelity DNA polymerase (Stratagene) was used as the polymerase for the PCR reaction, and the PCR was performed under conditions of denaturation at 95°C for 30 seconds; annealing at 55°C for 30 seconds; and polymerization at 72°C for 1 minute. The denaturation, annealing, and polymerization under such conditions were repeated 28 times. As a result, DNA fragments of 720 bp and 854 bp were obtained, respectively. The obtained DNA product was purified using a PCR Purification kit (QIAGEN). The purified amplification product and the pDC24 vector (SEQ ID NO: 16) treated with the restriction enzyme Smal and heat-treated at 65°C for 20 minutes were cloned using an Infusion Cloning Kit (TaKaRa) according to the provided manual, thereby constructing the vector pDC24△N1287(13032) for NCgl1287 deletion and target gene insertion.

**[Table 1]**

| SEQ ID NO | Name | Sequence |
|---|---|---|
| 1 | Primer | AATTCGAGCTCGGTACCCAATGGAGCTGAAAGAAT |
| 2 | Primer | CTTCCTGATAGTCCCGGGACATTGTTTTTC |
| 3 | Primer | GAAAAACAATGTCCCGGGACTATCAGGAAG |
| 4 | Primer | GTCGACTCTAGAGGATCCCCATAAAAACAGGCAGAGGA |

### Example 1-2: Construction of plasmid for introduction of pntAB

In order to further enhance the activity of the pntAB to be introduced, a plasmid was constructed in which the endogenous promoter of the *pntAB* gene to be introduced was replaced with PgapA.

First, PCR was performed using the chromosomal DNA of *Corynebacterium glutamicum* 13032 as a template with the primer pair of SEQ ID NO: 5 and SEQ ID NO: 6 to obtain a PgapA promoter fragment. In addition, the amino acid sequence of PntAB derived from *Corynebacterium variabile* and the nucleotide sequence encoding the same were obtained from NIH GenBank. Based on these sequences, the *pntAB* gene derived from *Corynebacterium variabile* was synthesized using the gene synthesis service of Cosmogenetech. PCR was performed using the synthesized *pntAB* gene as a template with the primers of SEQ ID NO: 7 and SEQ ID NO: 8, respectively. The primer sequences used to perform each of the PCR reactions are as shown in Table 2 below.

**[Table 2]**

| SEQ ID NO | Name | Sequence |
|---|---|---|
| 5 | Primer | CCTGAAAAACAATGTCCCAACGACCGAGCC TATTG |
| 6 | Primer | GTTGTGTCTCCTCTAAAGAT |
| 7 | Primer | TCTTTAGAGGAGACACAACATGCTTATTGGT ATTCCGAG |
| 8 | Primer | AACCAACTTCCTGATAGTCCCCTACAGGTGG |
| | | GAGATGATCT |

Pfu Ultra^{™} high-fidelity DNA polymerase (Stratagene) was used as the polymerase for the PCR reaction, and the PCR was performed under conditions of denaturation at 95°C for 30 seconds; annealing at 55°C for 30 seconds; and polymerization at 72°C for 1 minute. The denaturation, annealing, and polymerization under such conditions were repeated 28 times. As a result, a 472 bp DNA fragment of the PgapA promoter region and a 3041 bp DNA fragment of the *pntAB* gene of *Corynebacterium variabile* were obtained, respectively.

PCR was performed using the amplified promoter and the pntAB DNA fragment of *Corynebacterium variabile* as templates with the primer pair of SEQ ID NO: 5 and SEQ ID NO: 8. The PCR was performed under conditions of denaturation at 95°C for 5 minutes, followed by 28 cycles of denaturation at 95°C for 30 seconds, annealing at 55°C for 30 seconds, and polymerization at 72°C for 2 minutes, and then polymerization at 72°C for 5 minutes.

As a result, a pntAB DNA fragment of about 3.5 kb encoding the nicotinamide nucleotide transhydrogenase of *Corynebacterium variabile* linked to the PgapA promoter was amplified.

The amplification product was purified using a PCR Purification kit (QIAGEN) and used as an insert DNA fragment for vector construction. The purified amplification product and the pDC24△N1287(13032) vector treated with the restriction enzyme Smal and heat-treated at 65°C for 20 minutes were combined at a molar (M) ratio of 2:1, and cloned using an Infusion Cloning Kit (TaKaRa) according to the provided manual, thereby constructing the vector pDC24△N1287(13032)::PgapA_pntAB for introducing the *pntAB* gene of *Corynebacterium variabile* into the chromosome of *Corynebacterium glutamicum.*

### Example 2: Construction of strain with random mutations introduced in pntAB

### Example 2-1: Construction of plasmid library for introduction of random mutations in pntAB

Error-prone PCR was performed using a Diversify PCR Random Mutagenesis Kit (Takara) to induce random mutagenesis in the wild-type *pntAB*(*C.va*) gene encoding a nicotinamide nucleotide transhydrogenase protein. In order to select mutation rate conditions, error-prone PCR was performed under two conditions as described below, depending on the amount of MnSO₄ added. PCR was performed using pDC24△N1287(13032)::PgapA_pntAB as the DNA template for introducing mutations, and the primer pair of SEQ ID NO: 7 and SEQ ID NO: 8. PCR was performed under conditions of denaturation at 95°C for 30 seconds, followed by 25 cycles of denaturation at 95°C for 30 seconds, annealing at 55°C for 30 seconds, and polymerization at 68°C for 2 minutes; then a polymerization at 68°C for 3 minutes.

The composition for performing error-prone PCR is as shown in Table 3 below.

**[Table 3]**

| case # | case #1(MnSO₄ 1 µl) | case #2(MnSO₄ 2 µl) |
|---|---|---|
| 10X Titanium taq Buffer | 5 | 5 |
| MnSO₄(8mM) | 1 | 2 |
| dGTP (2mM) | 1 | 1 |
| 50 X dNTP Mix | 1 | 1 |
| Titanium Taq Polymerase | 1 | 1 |
| Forward primer(5pmol) | 2 | 2 |
| Reverse primer(5pmol) | 2 | 2 |
| Template DNA | 1 | 1 |
| dH₂O | 36 | 35 |
| Total | 50 | 50 |

The error-prone PCR product obtained above was treated with DpnI to remove the template plasmid, and the resulting DNA was cloned into the pDC24△N1287(13032) vector digested with the restriction enzyme SmaI using the Gibson assembly method, thereby obtaining the recombinant mutant plasmid library pDC24△N1287(13032)::PgapA_pntAB(mt).

### Example 2-2: Screening of randomly mutated pntAB library

The pntAB mutant plasmid library constructed in Example 2-1 and the control pDC24△N1287(13032)::PgapA_pntAB were transformed into *Corynebacterium glutamicum* KCCM 12502P (CA09-0903, Korean Patent Publication No. 10-2126951), a threonine-producing strain; and then the threonine production levels were analyzed.

Specifically, the control CA09-0903::△N1287::PgapA_pntAB((WT)) and CA09-0903::△N1287::PgapA_pntAB((mt)) libraries were each inoculated into a 96-Deep Well Plate-Dome (Bioneer) containing 400 µl of seed medium, and cultured in a plate shaking incubator (TAITEC) at 32°C and 12,000 rpm for about 120 hours.

The threonine concentrations of about 3,000 cultured samples were each measured by NIR, and pntAB mutations were examined by sequencing in six strains exhibiting increased threonine concentrations compared to the wild-type pntAB-expressing strain. As a result, these strains were shown to express PntAB having variant sequences of PntA(V437A)B, PntA(S476P)B(D14V), PntA(I306S)B, PntA(N41I)B, PntAB(E119G), or PntA(L342S, I486T)B(L199P, S346T).

### Example 3: Construction of L-amino acid-producing strain introduced with variant pntAB and Evaluation of amino acid producing ability

### Example 3-1: Construction of L-threonine-producing strain introduced with variant pntAB and Evaluation of threonine-producing ability

In order to confirm the threonine productivity of the six strains screened in Example 2-2 at the flask level, culturing was performed by the following method.

First, each strain was inoculated into a 250 ml corner-baffle flask containing 25 ml of seed medium, and shaking cultured at 200 rpm and 30°C for 20 hours. Then, 1 mL of the seed culture was inoculated into a 250 mL corner-baffle flask containing 24 mL of a production medium, and shaking cultured at 200 rpm and 32°C for 24 hours.

### <Seed Medium (pH 7.0)>

Glucose 20 g, Peptone 10 g, Yeast Extract 5 g, Urea 1.5 g, KH₂PO₄ 4 g, K₂HPO₄ 8 g, MgSO₄·7H₂O 0.5 g, Biotin 100 µg, Thiamine HCl 1000 µg, Calcium Pantothenate 2000 µg, and Nicotinamide 2000 µg (per 1 L of distilled water).

### <Production Medium (pH 7.2)>

Glucose 30 g, KH₂PO₄ 2 g, Urea 3 g, (NH₄)₂SO₄ 40 g, Peptone 2.5 g, Corn steep liquor (CSL, Sigma) 5 g (10 mL), MgSO₄·7H₂O 0.5 g, Leucine 400 mg, CaCO₃ 20 g (based on 1 L of distilled water)

After completion of the culture, the amount of L-threonine produced was measured using high-performance liquid chromatography (HPLC). The concentrations are shown in Table 4 below.

**[Table 4]**

| Threonine-producing ability of PntAB variant-expressing microorganism | | |
|---|---|---|
| Strain Name | L-Threonine Concentration (g/L) | Concentration Percentage (%) Relative to Wild-Type pntAB Strain |
| CA09-0903 ΔN1287::PgapA_pntAB | 5.6 | 100 |
| CA09-0903 ΔN1287::PgapA_pntA(V437A)B | 5.8 | 104 |
| CA09-0903 ΔN1287::PgapA_pntA(S476P)B(D14V) | 5.3 | 95 |
| CA09-0903 ΔN1287::PgapA_pntA(I306S)B | 5.0 | 89 |
| CA09-0903 ΔN1287::PgapA_pntA(N41I)B | 5.2 | 93 |
| CA09-0903 ΔN1287::PgapA_pntA(E119G) | 4.6 | 82 |
| CA09-0903 ΔN1287::PgapA_pntA(L342S, I486T)B(L199P, S346T) | 4.9 | 88 |

As a result, as shown in Table 4, it was confirmed that the parent strain *Corynebacterium glutamicum* CA09-0903 △N1287::PgapA_pntAB produced about 5.6 g/L of threonine. Among the strains selected in Example 2-2, it was confirmed that the threonine-producing ability of CA09-0903 △N1287::PgapA_pntA(V437A)B, a strain expressing PntAB(V437A), was increased by 4% compared to the control.

The above results suggest that a strain expressing pntA(V437A), in which the amino acid at position 437 of PntA in the amino acid sequence of PntAB derived from *Corynebacterium variabile* is modified (*e.g*., valine is replaced with alanine), exhibits increased pntAB activity compared to the wild-type, and is thus capable of producing an L-amino acid more efficiently.

Based on the above description, it will be understood by those skilled in the art that the present disclosure may be implemented in a different specific form without changing the technical spirit or essential characteristics thereof. In this regard, it should be understood that the foregoing examples are illustrative in all respects and are not to be construed as limiting. The scope of the present disclosure should be construed as including the meaning and scope of the appended claims rather than the detailed description, and all changes or variations derived from the equivalent concepts fall within the scope of the present disclosure.

## Claims

1. A variant polypeptide of a nicotinamide nucleotide transhydrogenase α-subunit, wherein the amino acid corresponding to position 437 of SEQ ID NO: 10 is substituted with a different amino acid.

2. The variant polypeptide according to claim 1, wherein the amino acid corresponding to position 437 of SEQ ID NO: 10 is substituted with alanine.

3. The variant polypeptide according to claim 1, wherein the amino acid sequence of the variant polypeptide has at least 90% identity to the amino acid sequence of SEQ ID NO: 10.

4. The variant polypeptide according to claim 1, wherein the variant polypeptide consists of the amino acid sequence of SEQ ID NO: 14.

5. A nicotinamide nucleotide transhydrogenase comprising the variant polypeptide of any one of claims 1 to 4.

6. A polynucleotide encoding the variant polypeptide of any one of claims 1 to 4.

7. A microorganism comprising at least one selected from the group consisting of the variant polypeptide of any one of claims 1 to 4, and a polynucleotide encoding the variant polypeptide.

8. The microorganism according to claim 7, wherein the microorganism is a microorganism of the genus *Corynebacterium*.

9. The microorganism according to claim 8, wherein the microorganism of the genus *Corynebacterium* is *Corynebacterium glutamicum*.

10. The microorganism according to claim 7, wherein the microorganism has an increased L-amino acid-producing ability as compared to a microorganism comprising a polypeptide of SEQ ID NO: 10 or a polynucleotide encoding the same.

11. The microorganism according to claim 10, wherein the L-amino acid is L-threonine.

12. A method for producing an L-amino acid, comprising culturing the microorganism according to claim 7 in a medium.

13. The method according to claim 12, further comprising recovering the L-amino acid from the cultured microorganism, a culture of the microorganism, a fermentation product of the microorganism, or the culture medium.

14. The method according to claim 13, wherein the L-amino acid is L-threonine.

15. Use of the variant polypeptide according to any one of claims 1 to 4, or a microorganism comprising the variant polypeptide or a polynucleotide encoding the variant polypeptide, for L-amino acid production.
